(19) Europäisches Patentamt
European Patent Office
Office européen des brevets

(11) Veröffentlichungsnummer: 0 351 641
A1

(12) # EUROPÄISCHE PATENTANMELDUNG

(21) Anmeldenummer: 89112245.9

(22) Anmeldetag: 05.07.89

(51) Int. Cl.[4]: C07D 207/325 , A01N 43/34 , C07D 209/44 , C07D 401/12 , C07D 413/12 , C07D 417/12 , C07D 403/12

(30) Priorität: 18.07.88 JP 177042/88
22.10.88 JP 266662/88

(43) Veröffentlichungstag der Anmeldung: 24.01.90 Patentblatt 90/04

(84) Benannte Vertragsstaaten: BE CH DE FR GB IT LI NL

(71) Anmelder: NIHON TOKUSHU NOYAKU SEIZO K.K.
7-1, Nihonbashi Honcho 2-Chome Chuo-ku
Tokyo(JP)

(72) Erfinder: Kume, Toyohiko
6-7-8, Asahigaoka
Hino-shi Tokyo(JP)
Erfinder: Goto, Toshio
3454-21, Honmachida
Machida-Shi Tokyo(JP)
Erfinder: Kamochi, Atsumi
2-24-10, Higashi-Toyoda
Hino-shi Tokyo(JP)
Erfinder: Yanagi, Akihiko
2-1200-1, Nagabuchi
Oume-shi Tokyo(JP)
Erfinder: Miyauchi, Hiroshi
39-15, Namiki-cho
Hachioji-shi Tokyo(JP)
Erfinder: Asami, Tadao
2-14-7, Sakuragaoka
Tama-shi Tokyo(JP)

(74) Vertreter: Schumacher, Günter, Dr. et al
c/o Bayer AG Konzernverwaltung RP
Patentabteilung
D-5090 Leverkusen 1 Bayerwerk(DE)

(54) 1-Phenylpyrrol.

(57) Offenbart werden neue 1-Phenylpyrrole der Formel (I)

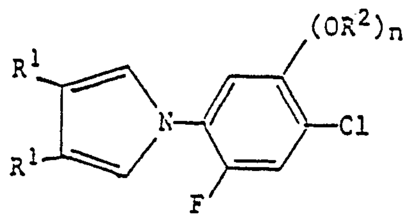

(I)

Verfahren zur Herstellung dieser neuen Verbindungen sowie deren Verwendung als Herbizide.

EP 0 351 641 A1

# 1-Phenylpyrrole

Die vorliegende Erfindung betrifft neue 1-Phenylpyrrole, Verfahren zu ihrer Herstellung und ihre Verwendung als Herbizide.

Bestimmte 1-Phenylpyrrole sind bereits bekannt {siehe Tetrahedron, Band 23, Nr. 11, Seiten 4467-4479 (1967); J. Chem. Soc. 1970, Nr. 18, Seiten 2563-2567; J. Heterocycl. Chem., Band 9, Nr. 6, Seiten 1413-1417 (1972) und Khim. Farm. Zh., Band 10, Nr. 9, Seiten 55-60 (1976)}, jedoch gibt es in den Literaturstellen keinerlei Offenbarung hinsichtlich einer herbiziden Funktion.

Nunmehr wurden neue 1-Phenylpyrrole der Formel (I)

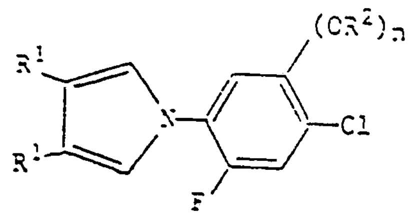

(I)

gefunden, in der

$R^1$ Methyl oder eine aus zwei miteinander verbundenen Resten $R^1$ gebildete Tetramethylen-Gruppe bezeichnet,

$R^2$ Wasserstoff, $C_{1-6}$-Alkyl, Cyclopropylmethyl, $C_{1-4}$-Alkoxy-$C_{1-2}$-alkyl, $C_{1-4}$-Alkylthio-$C_{1-2}$-alkyl, $C_{1-4}$-Alkylsulfinyl-$C_{1-2}$-alkyl, $C_{1-4}$-Alkylsulfonyl-$C_{1-2}$-alkyl oder $R^2$ Benzyl oder Phenethyl, die gegebenenfalls durch Halogen substituiert sind, weiterhin $R^2$ $C_{3-4}$-Alkenyl, $C_{3-4}$-Halogenoalkenyl, $C_{3-4}$-Alkinyl, $C_{2-3}$-Cyanoalkyl, Carbamoylmethyl, Thiocarbamoylmethyl, Trimethylsilylmethyl, $CH_2COOR^3$,

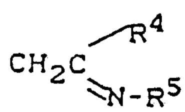

oder eine $C_{1-2}$-Alkyl-Gruppe, die mit einer gegebenenfalls substituierbaren 5- oder 6-gliedrigen heterocyclischen Gruppe verbunden ist, bezeichnet, wobei die fünfgliedrige heterocyclische Gruppe vorzugsweise ein Stickstoff-Atom und ein oder zwei weitere, aus Stickstoff, Schwefel und Sauerstoff ausgewählte Hetero-Atome enthält und wobei die sechsgliedrige heterocyclische Gruppe vorzugsweise ein oder zwei Stickstoff-Atome enthält, worin

$R^3$ $C_{1-6}$-Alkyl oder $C_{5-6}$-Cycloalkyl bezeichnet,

$R^4$ Wasserstoff, Methyl, Ethyl oder Cyclopropyl bezeichnet,

$R^5$ $OR^6$ oder $N(CH_3)_2$ bezeichnet, worin

$R^6$ Wasserstoff, $C_{1-5}$-Alkyl, Allyl, Propargyl oder Benzyl bezeichnet, und

n 0 oder 1 darstellt.

Die Verbindungen der Formel (I) können mittels eines Verfahrens erhalten werden, bei dem

a) Verbindungen der Formel (II)

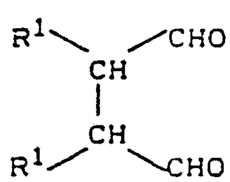

(II)

,

in der $R^1$ die gleichen Substituenten bezeichnet, wie sie im Vorstehenden genannt sind, mit Verbindungen der Formel (III)

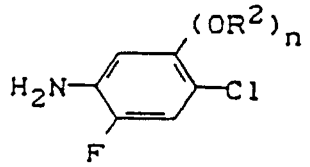

(III)

,

in der $R^2$ und n die gleichen Bedeutungen haben, wie sie im Vorstehenden angegeben sind, in Gegenwart inerter Lösungsmittel umgesetzt werden oder

b) Verbindungen der Formel (IV)

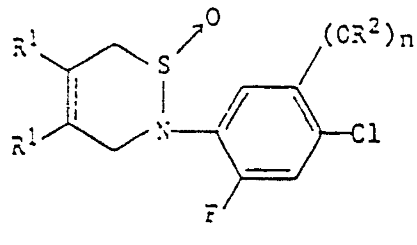

(IV)

,

in der $R^1$, $R^2$ und n die gleichen Bedeutungen haben, wie sie im Vorstehenden angegeben sind, einer Alkali-Behandlung in Gegenwart inerter Lösungsmittel unterworfen werden oder

c) in dem Fall, in dem $R^2$ nicht Wasserstoff ist, Verbindungen der Formel (V)

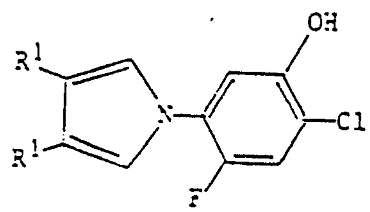

(V)

,

in der $R^1$ die gleichen Substituenten bezeichnet, wie sie im Vorstehenden genannt sind, mit Verbindungen der Formel (VI)

M - $R^{2-1}$ (VI),

in der $R^{2-1}$ die gleichen Substituenten wie $R^2$ mit Ausnahme von Wasserstoff bezeichnet und M Halogen, Methansulfonyloxy, Tosyloxy oder $R^{2-1}$-$OSO_2$-O bezeichnet, worin $R^{2-1}$ die im Vorstehenden angegebenen Bedeutungen hat, in Gegenwart inerter Lösungsmittel und gegebenenfalls in Gegenwart einer Base umgesetzt werden.

Die erfindungsgemäßen neuen 1-Phenylpyrrole zeigen potente herbizide Eigenschaften.

Aufgrund der Untersuchungen der Erfinder wurde überraschenderweise gefunden, daß beispielsweise im Vergleich zu bekannten Verbindungen, wie den in den oben genannten Veröffentlichungen offenbarten, die vorliegenden, durch die allgemeine Formel (I) bezeichneten 1-Phenylpyrrole im wesentlichen eine in hohem Maße ausgezeichnete herbizide Wirkung zeigen.

Unter den erfindungsgemäßen 1-Phenylpyrrolen der Formel (I) sind bevorzugte Verbindungen diejenigen, in denen

$R^1$ Methyl oder eine aus zwei miteinander verbundenen Resten $R^1$ gebildete Tetramethylen-Gruppe bezeichnet,

$R^2$ Wasserstoff, $C_{1-4}$-Alkyl, Cyclopropylmethyl, $C_{1-2}$-Alkoxy-$C_{1-2}$-alkyl, $C_{1-2}$-Alkylthio-$C_{1-2}$-alkyl, $C_{1-2}$-Alkylsulfinylmethyl, $C_{1-2}$-Alkylsulfonylmethyl, Benzyl, $C_3$-Alkenyl, $C_3$-Halogenoalkenyl, Propargyl, Cyanomethyl oder eine Methyl Gruppe, die mit einer fünfgliedrigen heterocyclischen Gruppe oder einer sechsgliedrigen aromatischen heterocyclischen Gruppe verbunden ist, bezeichnet, die jeweils wenigstens ein Stickstoffatom enthalten, wobei, betrachtet man den fünfgliedrigen Ring, vorzugsweise ein Stickstoff-Atom und ein oder zwei weitere, aus Stickstoff, Schwefel und Sauerstoff ausgewählte Hetero-Atome und, betrachtet man den sechsgliedrigen Ring, vorzugsweise ein oder zwei Stickstoff-Atome umfassen, wobei der fünfgliedrige und der sechsgliedrige Ring durch $C_{1-2}$-Alkyl oder $C_{1-2}$-Alkoxy substituiert sein können,

und

n 0 oder 1 darstellt.

Ganz besonders bevorzugte 1-Phenylpyrrole der Formel (I) sind diejenigen, in denen

$R^1$ Methyl oder eine aus zwei miteinander verbundenen Resten R gebildete Tetramethylen-Gruppe bezeichnet,

$R^2$ n-Propyl, iso-Propyl, n-, iso-, sec- oder tert-Butyl, Cyclopropylmethyl, Methoxymethyl, Ethoxymethyl, Methylthiomethyl, Ethylthiomethyl, Benzyl, Allyl, 2-Chloroallyl, Propargyl, Cyanomethyl oder eine mit einer fünfgliedigen heterocyclischen Gruppe, die ein oder zwei Stickstoff-Atome und entweder ein Sauerstoff- oder ein Schwefel-Atom umfaßt, verbundene Methyl-Gruppe oder eine mit einer sechsgliedigen aromatischen heterocyclischen Gruppe, die ein oder zwei Stickstoff-Atome umfaßt, vorzugsweise Pyridin oder Pyrimidin, verbundene Methyl-Gruppe bezeichnet, wobei der fünfgliedrige und der sechsgliedrige Ring durch eine Methyl-oder eine Methoxy-Gruppe substituiert sein können, und

n 1 darstellt.

In den im Vorstehenden genannten Definitionen für $R^2$ und den danach genannten Definitionen für $R^{2-1}$ können die fünfgliedrigen oder sechsgliedrigen heterocyclischen Ring-Substituenten als zu bevorzugende Beispiele Triazol, insbesondere 1,2,4-Triazol, Oxadiazol, insbesondere 1,2,5-Oxadiazol, Thiadiazol, insbesondere 1,2,5-Thiadiazol, Thiazol, Pyridin, Pyrimidin etc. umfassen.

Speziell erwähnt seien die folgenden Verbindungen:

2-(4-Chloro-2-fluoro-5-propargyloxyphenyl)-4,5,6,7-tetrahydroisobenzindol,

3,4-Dimethyl-1- (4-chloro-2-fluoro-5-propargyloxyphenyl)pyrrol,

2-(5-Allyloxy-4-chloro-2-fluoro-phenyl)-4,5,6,7-tetrahydroisobenzindol,

2-[4-Chloro-2-fluoro-5-(pyridin-2-yl-methoxy)phenyl]-4,5,6,7-tetrahydroisobenzindol und

2-[4-Chloro-2-fluoro-5-(isoxazol-3-yl-methoxy)phenyl]-4,5,6,7-tetrahydroisobenzindol.

Wenn als Ausgangsstoffe in dem Verfahren (a) beispielsweise 1,2-Cyclohexan-dicarboxyaldehyd und 4-Chloro-2-fluoro-5-propargyloxyanilin eingesetzt werden, kann die Reaktion durch das folgende Schema ausgedrückt werden:

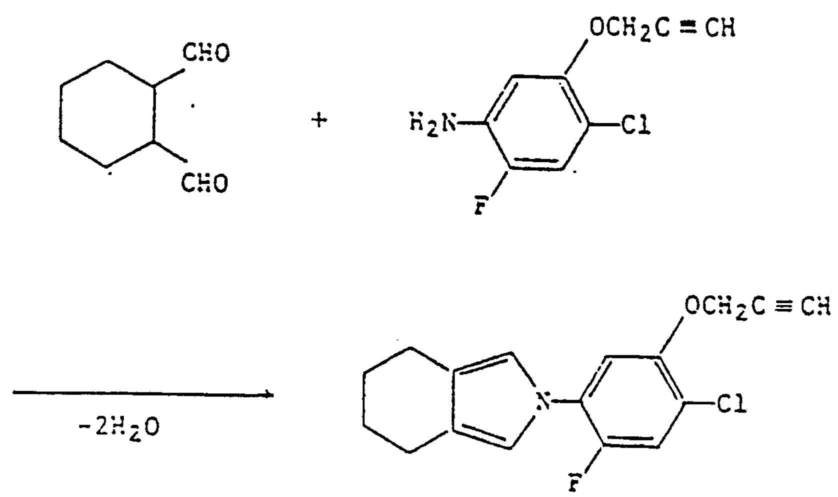


Wenn als Ausgangsstoffe in dem Verfahren (b) beispielsweise 4,5-Dimethyl-2-(4-chloro-2-fluoro-5-propargyloxyphenyl)-2,3-dihydro-6H-1,2-thiazin-1-oxid und Kaliumhydroxid eingesetzt werden, kann die Reaktion durch das folgende Schema ausgedrückt werden:

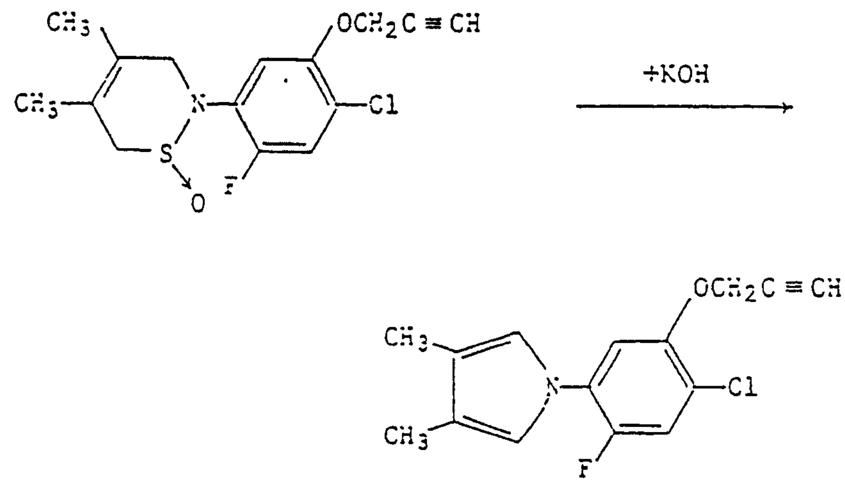


Wenn als Ausgangsstoffe in dem Verfahren (c) beispielsweise 3,4-Dimethyl-1-(4-chloro-2-fluoro-5-hydroxyphenyl)pyrrol und Bromoacetonitril eingesetzt werden, kann die Reaktion durch das folgende Schema ausgedrückt werden:

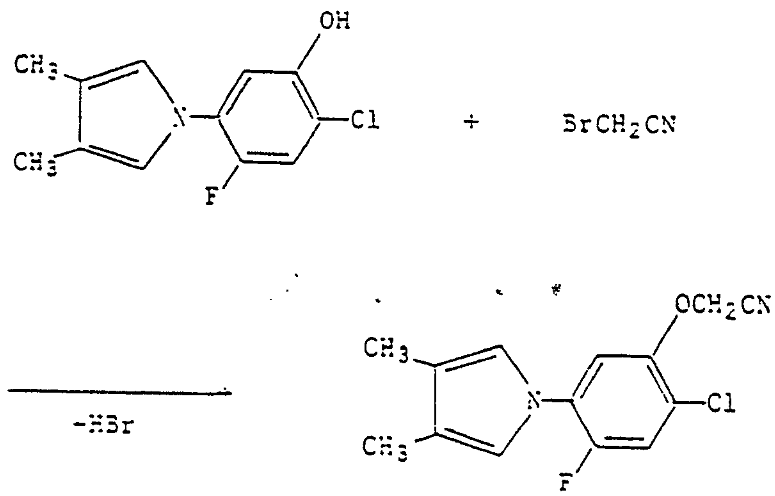


In den als Ausgangsstoffen bei dem Verfahren (a) eingesetzten Verbindungen der Formel (II) hat das Symbol $R^1$ die gleichen Bedeutungen, wie sie im Vorstehenden angegeben sind. Weiterhin hat in der Formel (II) das Symbol $R^1$ vorzugsweise die Bedeutungen, die im Vorstehenden als bevorzugt angegeben sind.

Die Verbindungen der Formel (II) selbst sind bekannt, und speziell 1,2-Cyclohexan-dicarboxaldehyd kann allgemein nach dem Verfahren hergestellt werden, das in Ann., Band 560, S. 1 (1948), offenbart ist, worin Cyclooctatetraen mittels einer Vier-Stufen-Reaktion in 7,8-Dihydroxybicyclo[4,2,0]octan überführt wird, das dann in dem abschließenden Reaktionsschritt mit Bleitetraacetat umgesetzt wird.

Weiterhin kann die oben genannte Verbindung (II) hergestellt werden mittels des Verfahrens, das in J. Amer. Chem. Soc., Div. Polymer Chem., Preprints, Band 5, Nr. 1, Seiten 210-215 (1964), offenbart ist, worin 1,2-Cyclohexan-dicarbonsäure in das entsprechende Dicarbonsäurechlorid überführt wird, das danach mit N-Methylanilin zu dem entsprechenden Bis-(N-methylanilid) umgesetzt wird, welches dann mit Lithiumaluminiumhydrid reduziert wird, oder mittels des Verfahrens, das in J. Amer. Chem. Soc., Band 74, Seiten 3014-3018 (1952), offenbart ist, worin aus 2,5-Dimethoxy-2,5-dihydrofuran und Butadien 4-Cyclohexen-1,2-dicarboxaldehyd gebildet wird, der dann katalytisch reduziert wird.

Außer nach den oben genannten drei bekannten Verfahren kann 1,2-Cyclohexan-dicarboxaldehyd nach dem folgenden Verfahren hergestellt werden, bei dem

(d) die Verbindung 1,2-Cyclohexandimethanol

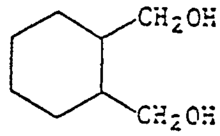

in Gegenwart inerter Lösungsmittel oxidiert wird.

Das vorerwähnte 1,2-Cyclohexandimethanol kann in einfacher Weise durch Reduktion von 1,2-Cyclohexan-dicarbonsäureanhydrid nach einem als solchem bekannten Verfahren hergestellt werden.

Als Oxidationsmittel in dem Verfahren (d) angewandt werden können beispielsweise Dimethylsulfoxid-Oxalsäuredichlorid {J. Org. Chem., Band 44, Seite 4148 (1979)}, Pyridiniumdichromat {Tetr. Letters 1979, Seite 399}, Pyridiniumchlorochromat {Tetr. Letters 1975, Seite 2647}, Mangandioxid, Sauerstoff, Bleitetraacetat, Kupferoxid, Ammoniumcer(IV)nitrat {Synthesis 1973, Seite 347}, Palladium(II)-Salz, Dimethylsulfoxid-Dicyclohexylcarbodiimid {J. Amer. Chem. Soc., Band 85, Seite 30277 (1963)} und so weiter.

Verglichen mit jedem der bekannten Verfahren erfordert das oben angegebene Verfahren (d) die minimale Anzahl von Schritten und sehr einfache Arbeitsweisen.

In den als Reaktionspartnern in dem Verfahren (a) eingesetzten Ausgangsstoffen der Formel (III) sind die Substituenten diejenigen, die im Vorstehenden für das Symbol $R^2$ angegeben sind.

In der allgemeinen Formel (III) haben die Symbole $R^2$ und n vorzugsweise die Bedeutungen, die im Vorstehenden als bevorzugt angegeben sind.

Die durch die allgemeine Formel (III) dargestellten Verbindungen sind bekannte Verbindungen, die in den EP-OSen 61 714 und 69 855 und der JP-OS 74639/1988 offenbart sind.

Zu Beispielen für die durch die allgemeine Formel (III) dargestellten Verbindungen zählen
4-Chloro-2-fluoro-5-propoxyanilin,
4-Chloro-5-cyclopropylmethoxy-2-fluoroanilin,
4-Chloro-2-fluoro-5-methoxymethoxyanilin,
4-Chloro-2-fluoro-5-methylthiomethoxyanilin,
4-Chloro-5-ethoxymethoxy-2-fluoroanilin,
4-Chloro-5-ethylthiomethoxy-2-fluoroanilin,
5-Benzyloxy-4-chloro-2-fluoroanilin,
5-Allyloxy-4-chloro-2-fluoroanilin,
4-Chloro-5-(2-chloroallyloxy)-2-fluoroanilin,
4-Chloro-2-fluoro-5-propargyloxyanilin,
4-Chloro-5-cyanomethoxy-2-fluoroanilin,
4-Chloro-2-fluoro-5-(isoxazol-3-yl-methoxy)anilin,
4-Chloro-2-fluoro-5-(4-methyl-1,2,5-oxadiazol-3-yl-methoxy)anilin,
4-Chloro-2-fluoro-5-(thiazol-4-yl-methoxy)anilin,
4-Chloro-2-fluoro-5-(pyridin-2-yl-methoxy)anilin
und so weiter.

In den als Ausgangsstoffe in dem oben angegebenen Verfahren (b) eingesetzten Ausgangsstoffen der Formel (IV) sind die Substituenten diejenigen, für die im Vorstehenden die Bedeutungen der Symbole $R^1$, $R^2$ und n angegeben sind.

In der allgemeinen Formel (IV) haben die Symbole $R^1$, $R^2$ und n vorzugsweise die Bedeutungen, die im Vorstehenden als bevorzugt angegeben sind.

Die durch die allgemeine Formel (IV) dargestellten Verbindungen können mittels des folgenden Verfahrens (e) erhalten werden, worin Verbindungen der allgemeinen Formel

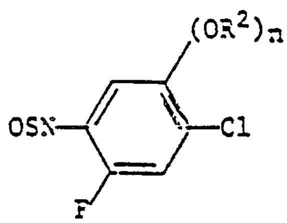 (VII)

in der $R^2$ die im Vorstehenden angegebenen Bedeutungen hat, mit 2,3-Dimethyl-1,3-butadien umgesetzt werden.

Die oben genannten, durch die allgemeine Formel (VII) dargestellten Verbindungen können mittels des folgenden Verfahrens (f) erhalten werden, worin die Verbindungen der oben genannten allgemeinen Formel (III) mit Thionylchlorid umgesetzt werden.

Die im Vorstehenden angegebenen Verfahren (e) und (f) werden konkret in den nachfolgenden Beispielen beschrieben, die allgemein nach der Verfahrensweise durchgefuhrt werden können, die in Collection Czechoslov. Chem. Communications, Band 19, Seiten 282-296 (1954), und in der gleichen Veröffentlichung, Band 19, Seiten 275-280 (1954), beschrieben ist.

Die in dem oben angegebenen Verfahren (c) eingesetzten Verbindungen der allgemeinen Formel (V) sind in den durch die allgemeine Formel (I) bezeichneten Verbindungen der vorliegenden Erfindung enthalten und können demgemäß mit Hilfe der oben bezeichneten Verfahren (a) und (b) hergestellt werden.

In den in dem oben angegebenen Verfahren (c) eingesetzten Verbindungen der Formel (VI) sind die Substituenten diejenigen, für die im Vorstehenden die Bedeutungen der Symbole $R^{2-1}$ und M angegeben sind, und vorzugsweise bezeichnet $R^{2-1}$ $C_{1-4}$-Alkyl Cyclopropylmethyl, $C_{1-2}$-Alkoxy-$C_{1-2}$-alkyl, $C_{1-2}$-Alkylthio-$C_{1-2}$-alkyl, Benzyl, $C_3$-Alkenyl, $C_3$-Halogenoalkenyl, Propargyl oder eine Cyanomethyl-Gruppe oder eine Methyl-Gruppe, die mit einer fünfgliedrigen heterocyclischen Gruppe oder einer sechsgliedrigen aromatischen heterocyclischen Gruppe verbunden ist, die jeweils wenigstens ein Stickstoffatom enthalten, wobei, betrachtet man den fünfgliedrigen Ring, vorzugsweise ein Stickstoff-Atom und ein oder zwei weitere, aus Stickstoff, Schwefel und Sauerstoff ausgewählte Hetero-Atome und, betrachtet man den sechsgliedrigen Ring, vorzugsweise ein oder zwei Stickstoff-Atome umfassen, wobei der fünfgliedrige und der sechsgliedrige Ring durch $C_{1-2}$-Alkyl oder $C_{1-2}$-Alkoxy substituiert sein können, und M bezeichnet Chlor, Brom, Iod, Methansulfonyloxy, Tosyloxy oder $R^{2-1}$-$OSO_2$-O, und $R^{2-1}$ bezeichnet die gleichen Substituenten, die im Vorstehenden als bevorzugt für das Symbol $R^{2-1}$ definiert sind.

Zu den Beispielen zählen Methyliodid, Dimethylsulfat, Ethyliodid, Isopropyliodid, Propyliodid, sec-Butyliodid, n-Butyliodid, Cyclopropylmethylbromid, Chloromethylmethylether, Chloromethylethylether, Chloromethylmethylsulfid, Chloromethylethylsulfid, Benzylchlorid, 2-Fluorobenzylchlorid, 4-Fluorobenzylchlorid, 2-Chlorobenzylchlorid, 4-Chlorobenzylchlorid, 3-Bromo-1-propen, 3-Bromo-2-methyl-1-propen, 2,3-Dichloro-1-propen, 1,3-Dichloro-1-propen, 1,2,3-Trichloro-1-propen, 1,1,2,3-Tetrachloro-1-propen, 3-Bromo-1-buten, 3-Bromo-1-propin, 3-Bromo-1-butin, Chloroacetonitril, 2-Bromopropionitril, Bromoacetonitril,
2-(Chloromethyl)pyridin,
2-(Chloromethyl)pyrazin,
1-(Chloromethyl)-1H-1,2,4-triazol,
5-(Chloromethyl)-1-methyl-1H-1,2,4-triazol,
3-(Chloromethyl)isoxazol,
3-(Chloromethyl)-5-methyl-1,2,4-oxadiazol,
5-(Chloroethyl)-3-methyl-1,2,4-oxadiazol,
3-(Bromomethyl)-4-methyl-1,2,5-oxadiazol,
3-(Chloromethyl)-5-methyl-1,3,4-oxadiazol,
4-Chloromethyl)thiazol,
3-(Chloromethyl)-5-methoxy-1,2,4-thiadiazol,
3-(Chloromethyl)-5-ethoxy-1,2,4-thiadiazol,
3-(Bromomethyl)-1,2,5-thiadiazol,
2-(Chloromethyl)-5-methyl-1,3,4-thiadiazol etc.

Als geeignete Verdünnungsmittel für die Durchführung des Verfahrens (a) können beliebige Arten von inerten organischen Lösungsmitteln genannt werden.

Als Beispiele für solche verwendbaren Lösungsmittel zu nennen sind vorzugsweise aliphatische Kohlenwasserstoffe wie beispielsweise Petrolether, Dichloromethan, Chloroform, Kohlenstofftetrachlorid, aromatische Kohlenwasserstoffe wie Benzol, Toluol und Xylol, Chlorobenzol und weitere, Ether wie beispielsweise Dimethoxyethan, 1,4-Dioxan und Tetrahydrofuran, Alkohole wie Methanol, Ethanol, Ethylcellosolve und Ethylenglycol und organische Säuren wie Essigsäure.

Die Reaktionstemperatur des Verfahrens (a) kann innerhalb eines Bereichs von beträchtlicher Breite variiert werden. Im allgemeinen wird die Reaktion bei einer Temperatur von etwa 50 °C bis etwa 200 °C, vorzugsweise bei einer Temperatur von etwa 80 °C bis etwa 150 °C, durchgeführt. Es wird bevorzugt, die Reaktion unter normalem Druck durchzuführen, obwohl auch ein höherer oder niedrigerer Druck angewandt werden kann.

Bei der Durchführung des Verfahrens (a) können beispielsweise etwa 1,3 mol der Verbindungen der Formel (III) auf 1 mol der Verbindungen der Formel (II) in Gegenwart eines inerten Lösungsmittels und, wie in den folgenden Beispielen angegeben ist, unter Erhitzen zum Rückfluß eingesetzt werden, so daß die angestrebten Verbindungen der Formel (I) erhalten werden können.

Bei der Durchführung des Verfahrens (b) ist es vorteilhaft, ein Verdünnungsmittel zu verwenden, darunter Alkohole wie Methanol und Ethanol.

Das Verfahren (b) kann bei einer Temperatur von etwa 50 °C bis etwa 150 °C durchgeführt werden.

Es wird bevorzugt, die Reaktion unter normalem Druck durchzuführen, wenngleich auch ein höherer oder niedrigerer Druck angewandt werden können.

Bei der Durchführung des Verfahrens (c) können inerte Lösungsmittel als geeignete Verdünnungsmittel verwendet werden.

Als Beispiele für solche verwendbaren Lösungsmittel zu nennen sind Wasser, Alkohole wie Methanol und Ethanol, Nitrile wie Acetonitril und Propionitril, Ether wie Tetrahydrofuran und 1,4-Dioxan, Kohlenwasserstoffe und halogenierte Kohlenwasserstoffe wie Petrolether, Chloroform, Kohlenstofftetrachlorid, Benzol, Toluol, Xylol und Chlorobenzol; Amide wie N,N-Dimethylformamid, Tetramethylharnstoff, N-Methyl-2-pyrrolidon und 1,3-Dimethyl-5-imidazolidon.

Weiterhin kann das Verfahren (c) auch in Gegenwart von Phasentransfer-Katalysatoren wie beispielsweise Trimethylbenzylammoniumchlorid, Tetrabutylammoniumbromid etc. durchgeführt werden.

Das Verfahren (c) kann auch in Gegenwart einer Base durchgeführt werden, beispielsweise einer Base, die aus der aus Natriumcarbonat, Kaliumcarbonat, Kaliumhydroxid, Natriumhydroxid, Natriummethoxid, Natriumethoxid, Kalium-tert-butoxid, Natriumhydrid etc. ausgewählt ist.

Die Reaktionstemperatur des Verfahrens (c) kann innerhalb eines Bereichs von beträchtlicher Breite variiert werden. Im allgemeinen wird die Reaktion bei einer Temperatur von etwa 10 °C bis etwa 150 °C, vorzugsweise bei einer Temperatur von etwa 40 °C bis etwa 100 °C, durchgeführt.

Es wird bevorzugt, die Reaktion unter normalem Druck durchzuführen, obwohl auch ein höherer oder niedrigerer Druck angewandt werden kann.

Bei der Durchführung des Verfahrens (c) können beispielsweise äquimolare Mengen bis zu etwa 1,2 mol der Verbindungen der allgemeinen Formel (VI) auf 1 mol der Verbindungen der allgemeinen Formel (V) eingesetzt werden, wobei die Reaktion in Gegenwart einer Base und eines inerten Lösungsmittels durchgeführt wird, um die angestrebten Verbindungen der Formel (I) zu erhalten.

Die erfindungsgemäßen aktiven Verbindungen können als Entlaubungsmittel, Abbrandmittel, Mittel zur Zerstörung breitblättriger Pflanzen und insbesondere als Unkrautvernichtungsmittel eingesetzt werden. Unter "Unkräutern" im weitesten Sinne sind alle Pflanzen zu verstehen, die an Stellen wachsen, wo sie nicht erwünscht sind. Ob die erfindungsgemäßen Substanzen als totale oder selektive Herbizide wirken, hängt im wesentlichen von der verwendeten Menge ab.

Die aktiven Verbindungen gemäß der Erfindung können beispielsweise in Verbindung mit den folgenden Pflanzen verwendet werden:

Dikotyledonen-Unkräuter der Gattungen:

Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver und Centaurea.

Dikotyledonen-Kulturen der Gattungen:

Gossypium, Glycine, Beta, Daucus, Phaseolus, Pisum, Solanum, Linum, Ipomoea, Vicia, Nicotiana, Lycopersicon, Arachis, Brassica, Lactuca, Cucumis und Cucurbita.

Monokotyledonen-Unkräuter der Gattungen:

Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus und Apera.

Monokotyledonen-Kulturen der Gattungen:

Oryza, Zea, Triticum, Hordeum, Avena, Secale, Sorghum, Panicum, Saccharum, Ananas, Asparagus und Allium.

Die Anwendung der erfindungsgemäßen aktiven Verbindungen ist jedoch keineswegs auf diese Gattungen begrenzt, sondern umfaßt auch andere Pflanzen in gleicher Weise.

In Abhängigkeit von den Konzentrationen können die Verbindungen zur totalen Bekämpfung von Unkräutern, beispielsweise auf Industriegelände und Bahnkörpern sowie auf Wegen und Plätzen mit oder ohne Baumbestand, verwendet werden. Ebenso können die Verbindungen auch zur Unkrautbekämpfung in perennierenden Kulturen, beispielsweise Aufforstungen, Zierbaumpflanzungen, Obstgärten, Weinbergen und -gärten, Zitrushainen, Nußbaumpflanzungen, Bananenplantagen, Kaffeeplantagen, Teeplantagen, Gummiplantagen, Ölpalmenplantagen, Kakaoplantagen, Weichfruchtplantagen und Hopfenfeldern, sowie zur selektiven Unkrautbekämpfung in Jahreskulturen verwendet werden.

Die Wirkstoffe können in übliche Präparate wie Lösungen, Emulsionen, benetzbare Pulver, Suspensionen, Pulver, Schäume, Pasten, Granulat, Aerosole, mit dem Wirkstoff imprägnierte natürliche und synthetische Materialien, sehr feine Kapseln in polymeren Substanzen, Beschichtungsmittel zum Aufbringen auf das Saatgut und Formulierungen, die in Verbindung mit Verbrennungseinrichtungen wie Räucherpatronen, Räucherdosen und Räucherschlangen eingesetzt werden, sowie ULV-Kaltvernebelungs- und Warmvernebelungspräparate umgewandelt werden.

Diese Präparate können in bekannter Weise hergestellt werden, beispielsweise durch Vermischen der Wirkstoffe mit Streckmitteln, d.h. flüssigen oder verflüssigten gasförmigen oder festen Verdünnungsmitteln oder Trägern, gegebenenfalls unter Verwendung von grenzflächenaktiven Mitteln, d.h. Emulgatoren und/oder Dispergiermitteln und/oder schaumbildenden Mitteln. Bei Verwendung von Wasser als Streckmittel können beispielsweise auch organische Lösungsmittel als Hilfslösungsmittel verwendet werden.

Als flüssige Verdünnungsmittel oder Träger eignen sich hauptsächlich aromatische Kohlenwasserstoffe wie Xylol, Toluol oder Alkylnaphthaline, chlorierte aromatische oder chlorierte aliphatische Kohlenwasserstoffe wie Chlorobenzole, Chloroethylene oder Methylenchlorid, aliphatische oder alicyclische Kohlenwasserstoffe wie Cyclohexan oder Paraffine, beispielsweise MineralölFraktionen, Alkohole wie Butanol oder Glycol sowie deren Ether und Ester, Ketone wie Aceton, Methylethylketon, Methylisobutylketon oder Cyclohexanon, oder stark polare Lösungsmittel wie Dimethylformamid oder Dimethylsulfoxid, sowie auch Wasser.

Unter verflüssigten gasförmigen Verdünnungsmitteln oder Trägern sind Flüssigkeiten zu verstehen, die bei normaler Temperatur und unter normalem Druck gasförmig sein würden, beispielsweise Aerosol-Treibmittel wie halogenierte Kohlenwasserstoffe sowie Butan, Propan, Stickstoff und Kohlenstoffdioxid.

Als feste Träger können gemahlene natürliche Minerale wie Kaoline, Tone, Talkum, Kreide, Quarz, Attapulgit, Montmorillonit oder Diatomeenerde, und gemahlene synthetische Minerale wie hochdisperse Kieselsäure, Aluminiumoxid und Silikate verwendet werden. Als feste Träger für Granulat können zerkleinertes und fraktioniertes natürliches Gesteinsmaterial wie Calcit, Marmor, Bimsstein, Sepiolith und Dolomit sowie synthetisches Granulat aus anorganischen und organischen Mehlen und Granulat aus organischem Material wie Sägemehl, Kokosnußschalen, Maiskolben und Tabakstengeln verwendet werden.

Als Emulgatoren und/oder Schaumbildner können nicht-ionische oder anionische Emulgatoren wie Polyoxyethylen-Fettsäureester, Polyoxyethylen-Fettalkoholether, beispielsweise Alkylarylpolyglycolether, Alkylsulfonate, Alkylsulfate, Arylsulfonate sowie Hydrolyseprodukte des Albumins verwendet werden. Zu den Dispergiermitteln zählen beispielsweise Ligninsulfit-Ablaugen und Methylcellulose.

Haftmittel wie Carboxymethylcellulose und natürliche und synthetische Polymerisate in Form von Pulver, Granulat oder Latices, z.B. Gummi arabicum, Polyvinylalkohol und Polyvinylacetat, können in den Formulierungen verwendet werden.

Es ist möglich, farbgebende Stoffe wie anorganische Pigmente, beispielsweise Eisenoxid, Titanoxid und Preußischblau, und organische Farbstoffe wie Alizarin-Farbstoffe, Azo-Farbstoffe oder Metallphthalocyanin-Farbstoffe, und Spurennährstoffe wie Salze von Eisen, Mangan, Bor, Kupfer, Cobalt, Molybdän und Zink, zu verwenden.

Die Präparate enthalten im allgemeinen von 0,1 bis 95 Gewichts-%, vorzugsweise von 0,5 bis 90 Gewichts-% des Wirkstoffs.

Die Wirkstoffe gemäß der vorliegenden Erfindung können als solche oder in Form ihrer Präparate auch zur Unkrautbekämpfung als Mischungen mit anderen Herbiziden eingesetzt werden, wobei Fertigpräparate oder Tankmischungen möglich sind.

Mischungen mit anderen aktiven Verbindungen wie Herbiziden, Fungiziden, Insektiziden, Akariziden, Nematiziden, vogelabweisenden Mitteln, Pflanzennährstoffen sowie Mitteln zur Verbesserung der Bodenstruktur sind ebenfalls möglich.

Die Wirkstoffe können als solche, in Form von Präparaten oder in Gebrauchsformen, die daraus durch weitere Verdünnung hergestellt werden, eingesetzt werden, beispielsweise in Form gebrauchsfertiger Lösungen, Suspensionen, Emulsionen, Pulver, Pasten und als Granulat. Sie werden in üblicher Weise angewandt, beispielsweise durch Bewässern, Sprühen, Zerstäuben oder Streuen.

Die erfindungsgemäßen Wirkstoffe können vor oder nach dem Auflaufen der Pflanzen zur Anwendung gelangen.

Sie können auch vor der Einsaat in den Boden eingearbeitet werden. Insbesondere werden sie nach dem Auflaufen der Pflanzen angewandt.

Die eingesetzten Mengen der aktiven Verbindung können innerhalb eines Bereichs von erheblicher Breite variiert werden. Sie hängen wesentlich von der Art der angestrebten Wirkung ab. Im allgemeinen liegen die aufgewandten Mengen des Wirkstoffs zwischen 0,0001 und 3 kg/ha, vorzugsweise zwischen 0,001 und 2 kg/ha.

Die Herstellung und Verwendung der erfindungsgemäßen Verbindungen sind den folgenden Beispielen zu entnehmen.

## Herstellungsbeispiele

### Beispiel 1

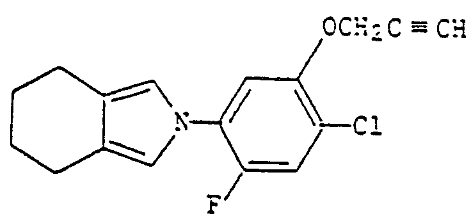

1,4 g 1,2-Cyclohexandicarboxyaldehyd und 2,2 g 4-Chloro-2-fluoro-5-propargyloxyanilin wurden zu 50 ml Xylol hinzugefügt, und die Mischung wurde 1 h zum Rückfluß erhitzt. Das Xylol wurde aus der Lösung unter vermindertem Druck abdestilliert, und der verbliebene Rückstand wurde über eine Silicagel-Säule (Hexan:Ethylacetat = 9:1) gereinigt, wonach 2,2 g des angestrebten 2-(4-Chloro-2-fluoro-5-propargyloxyphenyl)-4,5,6,7-tetrahydroisobenzindols erhalten wurden; $n_D^{20}$ 1,5938.

### Beispiel 2

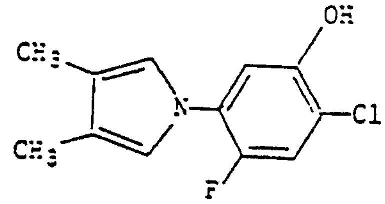

Zu einer Lösung von 4,39 g 4-Chloro-2-fluoro-5-methoxycarbonyloxyanilin, das in 40 ml Toluol gelöst worden war, wurden 2,86 g Thionylchlorid hinzugefügt, und die Mischung wurde anschließend 1 h zum Rückfluß erhitzt. Nach Beendigung der Reaktion wurden die Überschuß-Menge Thionylchlorid und das Lösungsmittel unter vermindertem Druck von dem Reaktionsprodukt abdestilliert. Der erhaltene Rückstand wurde in 40 ml Toluol gelöst, und 2,46 g 2,3-Dimethyl-1,3-butadien wurden hinzugefügt. Die Mischung wurden anschließend 5 h zum Rückfluß erhitzt. Das Lösungsmittel wurde unter vermindertem Druck abdestilliert. Der Rückstand wurde in 60 ml Ethanol gelöst, und der unlösliche Anteil wurde durch Filtration entfernt. Das Filtrat wurde zu etwa 100 ml einer Ethanol-Lösung von 3,7 g Kaliumhydroxid hinzugefügt, und die Mischung wurde. 1 h zum Rückfluß erhitzt. Nach Beendigung der Reaktion wurde das Lösungsmittel unter vermindertem Druck abdestilliert. Eine kleine Menge Wasser wurde zu dem Reaktionsprodukt

gegeben, das nach dem Neutralisieren mit Salzsäure zweimal mit Dichloromethan (jeweils 50 ml) extrahiert wurde. Die Extrakte wurden über wasserfreiem Magnesiumsulfat getrocknet und durch Destillation unter vermindertem Druck vom Lösungsmittel befreit. Der Rückstand wurde durch Silicagel-Säulenchromatographie gereinigt, wonach 2,42 g 3,4-Dimethyl-1-(4-chloro-2-fluoro-5-hydroxyphenyl)pyrrol mit einem Schmelzpunkt im Bereich von 84-85 °C erhalten wurden.

Beispiel 3

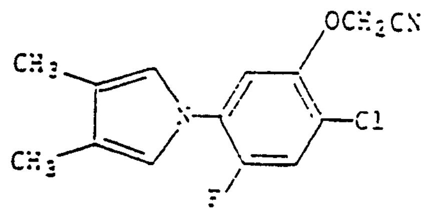

0,96 g 3,4-Dimethyl-1-(4-chloro-2-fluoro-5-hydroxyphenyl)pyrrol, 0,58 g Bromoacetonitril und 0,55 g pulverförmiges wasserfreies Kaliumcarbonat in 40 ml Acetonitril wurden 4 h erhitzt. Nach Beendigung der Reaktion wurde das Lösungsmittel unter vermindertem Druck aus der Reaktionsmischung abdestilliert. Eine kleine Menge Wasser wurde dem Rückstand zugesetzt, der danach zweimal mit Dichloromethan (jeweils 30 ml) extrahiert wurde.

Aus dem Extrakt wurde nach dem Trocknen mit wasserfreiem Kaliumcarbonat das Lösungsmittel unter vermindertem Druck abdestilliert. Der Rückstand wurde durch Silicagel-Säulenchromatographie gereinigt, wonach 0,88 g des angestrebten 3,4-Dimethyl-1-(4-chloro-5-cyanomethoxy-2-fluorophenyl)pyrrols erhalten wurden; $n_D^{20}$ 1,5792.

Beispiel 4 (Synthese eines Ausgangsstoffes)

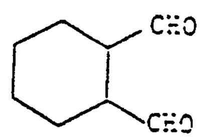

Zu einer Lösung von 20 ml Oxalylchlorid in 300 ml Dichloromethan wurde tropfenweise unter Rühren eine Lösung von 34 ml Dimethylsulfoxid in 100 ml Dichloromethan bei einer Temperatur von -70 °C hinzugefügt, wobei die Temperatur der Reaktionslösung unterhalb von -55 °C gehalten wurde. Nach Beendigung des Zutropfens wurde die Reaktionslösung 5 min gerührt, und danach wurde während eines Zeitraums von 5 min eine Lösung von 14,4 g cis-1,2-Cyclohexan-dimethanol in 100 ml Dichloromethan hinzugefügt. Nach 15 min Rühren wurden 140 ml Triethylamin zu der Lösung hinzugefügt, und die Mischung wurde stehen gelassen, bis sie wieder die Raumtemperatur angenommen hatte. Danach wurden 300 ml Wasser zu der Reaktionslösung gegeben, und anschließend wurde die Dichloromethan-Schicht davon abgenommen; die wäßrige Schicht wurde mit 200 ml Dichloromethan gewaschen, und die erhaltenen organischen Schichten wurden vereinigt und mit einer gesättigten Natriumchlorid-Lösung gewaschen. Die organische Schicht wurde mit wasserfreiem Natriumsulfat getrocknet und unter vermindertem Druck konzentriert. Der resultierende Rückstand wurde unter vermindertem Druck destilliert, und durch Sammeln der resultierenden, im Temperaturbereich von 85-90 °C bei 6,67 mbar (5 mmHg) siedenden Fraktionen wurden 11,8 g des angestrebten 1,2-Cyclohexandicarboxyaldehyds erhalten.

Bezugsbeispiel

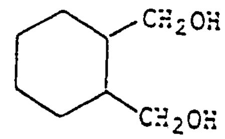

63 g wasserfreie cis-1,2-Cyclohexandicarbonsäure wurden in 200 ml getrocknetem Ether gelöst, und die Lösung wurde tropfenweise zu einer eisgekühlten Suspension von 29 g Lithiumaluminiumhydrid in 1000 ml getrocknetem Ether hinzugefügt. Nach Beendigung des Zutropfens wurde die Lösung 30 min zum Rückfluß erhitzt. Nach dem Abkühlen auf Raumtemperatur wurde die Lösung mit 50 ml Wasser versetzt, und die Mischung wurde filtriert. Der Filtrationsrückstand wurde zweimal mit Tetrahydrofuran (jeweils 200 ml) gewaschen, und das Filtrat wurde unter vermindertem Druck konzentriert. Der Rückstand wurde unter vermindertem Druck destilliert, und die bei 122-125 °C unter 0.93 mbar (0.7 mmHg) siedenden Fraktionen wurden gesammelt, wonach 53 g reines cis-1,2-Cyclohexandimethanol erhalten wurden.

Nach den gleichen Arbeitsweisen, wie sie in den vorhergehenden Beispielen 1 bis 3 angewandt wurden, wurden die nachstehenden Verbindungen der allgemeinen Formel (I) der vorliegenden Erfindung erhalten, die zusammen mit den in den Beispielen 1 bis 3 erhaltenen Verbindungen in Tabelle 1 aufgeführt sind.

## Tabelle 1

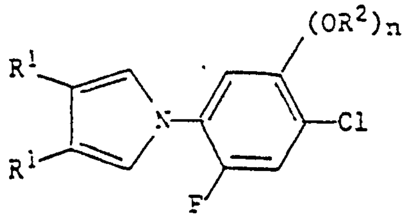

| Verbindung Nr. | $R^1$ | $(OR^2)_n$ | Schmelzpunkt (°C) oder $n_D^{20}$ |
|---|---|---|---|
| 1 | $-(CH_2)_4-$ | H | $n_D^{50}$ 1.5851 |
| 2 | $CH_3$ | OH | Schmp. 84 - 85°C |
| 3 | $-(CH_2)_4-$ | OH | |
| 4 | $-(CH_2)_4-$ | $OCH_3$ | |
| 5 | $-(CH_2)_4-$ | $OC_2H_5$ | |
| 6 | $CH_3$ | $OC_3H_7$-iso | $n_D^{20}$ 1.5600 |
| 7 | $-(CH_2)_4-$ | $OC_3H_7$-iso | |
| 8 | $CH_3$ | $OC_3H_7$-n | Öl |
| 9 | $-(CH_2)_4-$ | $OC_3H_7$-n | Öl |

| Verbindung Nr. | $R^1$ | $(OR^2)_n$ | Schmelzpunkt (°C) oder $n_D^{20}$ |
|---|---|---|---|
| 10 | $-(CH_2)_4-$ | $OC_4H_9$-sec | |
| 11 | $-(CH_2)_4-$ | $OC_4H_9$-n | |
| 12 | $-(CH_2)_4-$ | $O-CH_2-$ 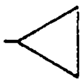 | |
| 13 | $-(CH_2)_4-$ | $OC_5H_{11}$-n | |
| 14 | $-(CH_2)_4-$ | $OC_6H_{13}$-n | |
| 15 | $-(CH_2)_4-$ | $OCH_2-$ 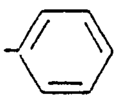 | |
| 16 | $-(CH_2)_4-$ | $OCH_2-$ 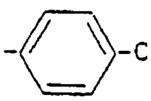 -Cl | |
| 17 | $-(CH_2)_4-$ | $OCH_2CH=CH_2$ | |
| 18 | $-(CH_2)_4-$ | $OCH_2C(Cl)=CH_2$ | |
| 19 | $-(CH_2)_4-$ | $OCH_2CH=CHCl$ | |
| 20 | $-(CH_2)_4-$ | $OCH_2C(Cl)=CHCl$ | |
| 21 | $-(CH_2)_4-$ | $OCH_2C(Cl)=CCl_2$ | |
| 22 | $CH_3$ | $OCH_2C\equiv CH$ | klebriges Öl |
| 23 | $-(CH_2)_4-$ | $OCH_2C\equiv CH$ | $n_D^{20}$ 1.5935 |

| Verbindung Nr. | $R^1$ | $(OR^2)_n$ | Schmelzpunkt (°C) oder $n_D^{20}$ |
|---|---|---|---|
| 24 | $-(CH_2)_4-$ | $OCH_2OCH_3$ | |
| 25 | $-(CH_2)_4-$ | $OCH_2OC_2H_5$ | |
| 26 | $-(CH_2)_4-$ | $OCH_2SCH_3$ | |
| 27 | $-(CH_2)_4-$ | $OCH_2SC_2H_5$ | |
| 28 | $CH_3$ | $OCH_2CN$ | $n_D^{20}$ 1.5792 |
| 29 | $-(CH_2)_4-$ | $OCH_2CN$ | Öl |
| 30 | $-(CH_2)_4-$ | $CH_3$<br>\|<br>OCHCN | |
| 31 | $CH_3$ | $OCH_2$- 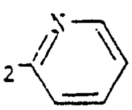 | Schmp. 125 - 126.5°C |
| 32 | $-(CH_2)_4-$ | $OCH_2$- 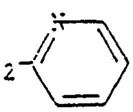 | Schmp. 110 - 113°C |
| 33 | $CH_3$ | $OCH_2$- 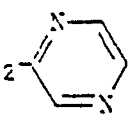 | |
| 34 | $-(CH_2)_4-$ | $OCH_2$- 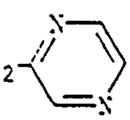 | |
| 35 | $CH_3$ | $OCH_2$- 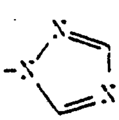 | |
| 36 | $-(CH_2)_4-$ | $OCH_2$- 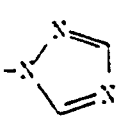 | |

| Verbindung Nr. | $R^1$ | $(OR^2)_n$ | Schmelzpunkt (°C) oder $n_D^{20}$ |
|---|---|---|---|
| 37 | $CH_3$ | 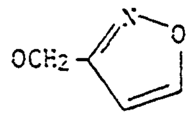 | Schmp. 91 - 97°C |
| 38 | $-(CH_2)_4-$ | 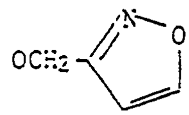 | Schmp. 90 - 100°C |
| 39 | $CH_3$ | 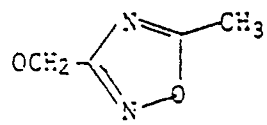 | |
| 40 | $O(CH_2)_4-$ | 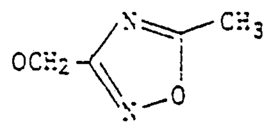 | |
| 41 | $CH_3$ | 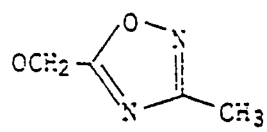 | Schmp. 68 - 71°C |
| 42 | $-(CH_2)_4-$ | 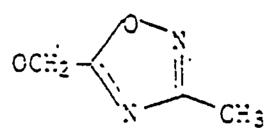 | |
| 43 | $CH_3$ | 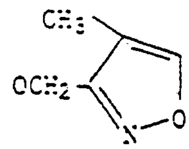 | |
| 44 | $-(CH_2)_4-$ | 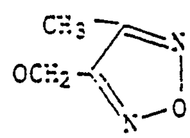 | |
| 45 | $CH_3$ | 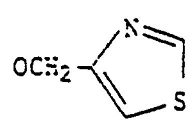 | |
| 46 | $-(CH_2)_4-$ | 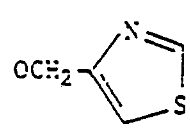 | |

| Verbindung Nr. | $R^1$ | $(OR^2)_n$ | Schmelzpunkt (°C) oder $n_D^{20}$ |
|---|---|---|---|
| 47 | $CH_3$ | 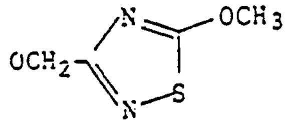 | |
| 48 | $-(CH_2)_4-$ | 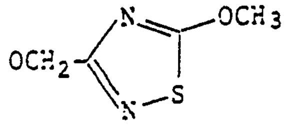 | |
| 49 | $CH_3$ | 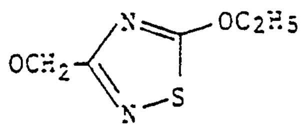 | |
| 50 | $-(CH_2)_4-$ | 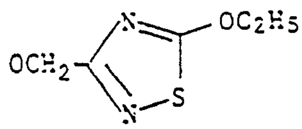 | |
| 51 | $CH_3$ | 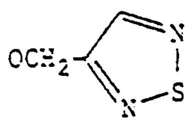 | |
| 52 | $-(CH_2)_4-$ | 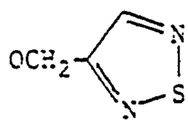 | |
| 53 | $CH_3$ | 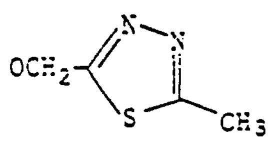 | |
| 54 | $-(CH_2)_4-$ | 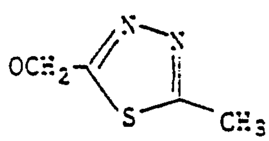 | |
| 55 | $-(CH_2)_4-$ | $OCH_2\overset{O}{\underset{O}{S}}-CH_3$ | Schmp. 105 - 114°C |
| 56 | $-(CH_2)_4-$ | $OCH_2\overset{O}{C}NH_2$ | |

| Verbindung Nr. | $R^1$ | $(OR^2)n$ | Schmelzpunkt (°C) oder $n_D^{20}$ |
|---|---|---|---|
| 57 | $-(CH_2)_4-$ | S<br>‖<br>$OCH_2CNH_2$ | |
| 58 | $-(CH_2)_4-$ | $OCH_2Si(CH_3)_3$ | |
| 59 | $-(CH_2)_4-$ | $OCH_2COOC_2H_5$ | öl |
| 60 | $-(CH_2)_4-$ | $OCH_2COO-$ 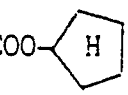 | |
| 61 | $-(CH_2)_4-$ | O<br>‖<br>$OCH_2CCH_3$ | |
| 62 | $-(CH_2)_4-$ | $OCH_2CH=N-OCH_3$ | |
| 63 | $-(CH_2)_4-$ | $OCH_2C=N-OH$<br>$CH_3$ | |
| 64 | $-(CH_2)_4-$ | $OCH_2C=N-OH$<br>$CH_3$ | |
| 65 | $-(CH_2)_4-$ | $N-OCH_2CH=CH_2$<br>$OCH_2C$<br>$CH_3$ | |
| 66 | $-(CH_2)_4-$ | $N-OCH_2-$ 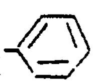<br>$OCH_2C$<br>$CH_3$ | |
| 67 | $-(CH_2)_4-$ | O<br>‖<br>$CH_2S-CH_3$ | |
| 68 | $-(CH_2)_4-$ | $CH_3$<br>\|<br>$CH-C≡CH$ | |

Biologische Test-Beispiele

Kontroll-Verbindungen

E-1

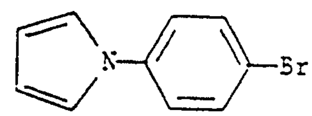

{offenbart in J. Chem. SoC. 1970, Nr. 18, Seiten 2563-2567}

E-2

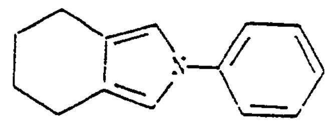

{offenbart in Khim. Farm. Zh., Band 10, Nr. 9, Seiten 55-60 (1976)}.

Beispiel 5

Test gegen Unkräuter in einem überfluteten Reisfeld durch Wasseroberflächen-Anwendung

Herstellung eines Wirkstoff-Präparats

Träger: 5 Gewichtsteile Aceton;
Emulgator: 1 Gewichtsteil Benzyloxypolyglycolether.

Ein Wirkstoff-Präparat in Form eines emulgierbaren Konzentrats wurde erhalten durch Vermischen von 1 Gewichtsteil der aktiven Verbindung mit den oben angegebenen Mengen des Trägers und des emulgierenden Mittels. Eine vorher festgelegte Menge des Präparats wurde mit Wasser verdünnt.

Test-Verfahren

Reis-Kulturboden wurde in Töpfe (5 $dm^2$; 25 cm x 20 cm x 9 cm) gefüllt, und Reis-Setzlinge (Varietät: "Nihonbare") im 2,5-Blattstadium (15 cm hoch) wurden jeweils an zwei Stellen pro Topf als Stock von drei Setzlingen umgepflanzt. Samen von Hühnerhirse (Echinochloa oryzicola Vasing.), Schirmkraut (Cyperus difformis L.), Monochoria (Monochoria vaginalis) sowie den einjährigen breitblättrigen Unkräutern Falsche Pimpernelle (Lindernia pyxidaria L.), Rotala indica, Amerikanische Wasserwurz (Elatine triandra), Rotstengel (Ammannia multiflora Roxburgh) und Dopatrium junceum Hamilton wurden eingesät, und die Töpfe wurden feucht gehalten. Zwei Tage später wurden die Töpfe bis zu einer Tiefe von etwa 2 bis 3 cm überflutet. Fünf Tage nach dem Umpflanzen der Setzlinge wurde die Verbindung der vorliegenden Erfindung, in Form eines wie im Vorstehenden hergestellten emulgierbaren Konzentrats, mittels einer Pipette in einer vorher festgelegten Menge auf die Oberfläche des Wassers aufgebracht. Danach wurde der Zustand der etwa 3 cm tiefen Überflutung aufrechterhalten, und vier Wochen nach der chemischen Behandlung wurden die herbizide Wirkung und die Phytotoxizität gegenüber Reis untersucht und mit Hilfe der folgenden Skala von 0 bis 5 bewertet:

| Bewertung | Herbizide Wirkung (bewertet als Unkrautvernichtungs-Verhältnis, bezogen auf eine unbehandelte Fläche) |
|---|---|
| 5 | wenigstens 95 % (verdorrt) |
| 4 | wenigstens 80 %, jedoch weniger als 95 % |
| 3 | wenigstens 50 %, jedoch weniger als 80 % |
| 2 | wenigstens 30 %, jedoch weniger als 50 % |
| 1 | wenigstens 10 %, jedoch weniger als 30 % |
| 0 | weniger als 10 % (unwirksam) |
| Bewertung | Phytotoxizität gegenüber Nutzpflanzen (bewertet unter Bezug auf die unbehandelte Fläche) |
| 5 | wenigstens 90 % (Ausrottung) |
| 4 | wenigstens 50 %, jedoch weniger als 90 % |
| 3 | wenigstens 30 %, jedoch weniger als 50 % |
| 2 | wenigstens 10 %, jedoch weniger als 30 % |
| 1 | mehr als 0 %, jedoch weniger als 10 % |
| 0 | 0 % (keine Phytotoxizität) |

Die Ergebnisse sind in Tabelle 2 aufgeführt.

Tabelle 2

| Aktive Verbindung Nr. | Wirkstoff-Menge kg/h | **Herbizide Wirkung** gegen Unkräuter | | | | Phytotoxizität gegen Reis-Pflanzen |
|---|---|---|---|---|---|---|
| | | Hühnerhirse | Schirmkraut | Monochoria | Breitblättrige Unkräuter | |
| 22 | 0,5 | 5 | 5 | 5 | 5 | 0 |
| | 0,25 | 4 | 5 | 5 | 5 | 0 |
| 23 | 0,5 | 5 | 5 | 5 | 5 | 1 |
| | 0,25 | 5 | 5 | 5 | 5 | 0 |
| Bekannte Verbindung | | | | | | |
| E-1 | 1,0 | 0 | 0 | 0 | 0 | 0 |
| E-2 | 1,0 | 0 | 0 | 0 | 0 | 0 |

Beispiel 6

Test gegen Unkräuter auf höher gelegenem Ackerboden durch Bodenbehandlung vor dem Auflaufen:

In einem Gewächshaus wurden Sojabohnen-Samen in Töpfe von 500 $cm^2$ Fläche, die mit Ackererde gefüllt waren, eingesät, und Erde, die Samen von Digitaria (Digitaria sanguinalis), grauem Amaranth (Amaranthus lividus L.) und Gänsefuß (Chenopodium album L.) enthielt, wurde über die Erde in den Töpfen in einer Tiefe von 1 cm gestreut.

Einen Tag nach der Einsaat wurde eine Test-Chemikalie in einer vorher festgelegten Konzentration, hergestellt wie in Beispiel 5 gleichmäßig über die Oberflächenschicht des Bodens in jedem der Test-Töpfe gesprüht.

Vier Wochen nach dem Sprühen wurden die herbizide Wirkung und die Phytotoxizität gegenüber den Nutzpflanzen anhand der gleichen Bewertungsskalen wie in Beispiel 5 geprüft. Die Ergebnisse sind in

Tabelle 3 aufgeführt.

Tabelle 3

| Aktive Verbindung Nr. | Wirkstoff-Menge kg/ha | **Herbizide Wirkung** gegen Unkräuter | | | Phytotoxizität gegen Sojabohnenpflanzen |
|---|---|---|---|---|---|
| | | Digitaria | Grauer Amaranth | Gänsefuß | |
| 6 | 1,0 | 3 | 5 | 5 | 0 |
| 22 | 0,5 | 4 | 5 | 5 | 0 |
| | 0,25 | 3 | 5 | 5 | 0 |
| 23 | 0,5 | 5 | 5 | 5 | 0 |
| | 0,25 | 4 | 5 | 5 | 0 |
| Bekannte Verbindung | | | | | |
| E-1 | 1,0 | 0 | 0 | 0 | 0 |
| E-2 | 1,0 | 0 | 0 | 0 | 0 |

Beispiel 7

Test gegen Unkräuter auf höher gelegenem Ackerboden durch Laub-Behandlung:

In einem Gewächshaus wurden Mais-Samen in Töpfe von 500 $cm^2$ Fläche, die mit Ackererde gefüllt waren, eingesät, und Erde, die Samen von Digitaria (Digitaria sanguinalis), grauem Amaranth (Amaranthus lividus L.) und Gänsefuß (Chenopodium album L.) enthielt, wurde über die Erde in den Töpfen in einer Tiefe von 1 cm gestreut.

Nach der Einsaat wurden die Pflanzen 14 Tage angezogen, und eine Test-Chemikalie in einer vorher festgelegten Konzentration, hergestellt wie in Beispiel 5, wurde gleichmäßig über Test-Pflanzen in jedem der Test-Töpfe gesprüht.

Vier Wochen nach dem Sprühen wurden die herbizide Wirkung und die Phytotoxizität gegenüber den Nutzpflanzen anhand der gleichen Bewertungsskalen wie in Beispiel 5 geprüft. Die Ergebnisse sind in Tabelle 4 aufgeführt.

Tabelle 5

| Aktive Verbindung Nr. | Wirkstoff-Menge kg/ha | **Herbizide Wirkung** gegen Unkräuter | | | Phytotoxizität gegen Maispflanzen |
|---|---|---|---|---|---|
| | | Digitaria | Grauer Amaranth | Gänsefuß | |
| 22 | 1,0 | 5 | 5 | 5 | 1 |
| | 0,5 | 4 | 5 | 4 | 0 |
| 23 | 0,5 | 4 | 5 | 5 | 1 |
| | 0,25 | 3 | 5 | 4 | 0 |
| Bekannte Verbindung | | | | | |
| E-1 | 1,0 | 0 | 0 | 0 | 0 |
| E-2 | 1,0 | 0 | 0 | 0 | 0 |

## Ansprüche

1. 1-Phenylpyrrole der Formel (I)

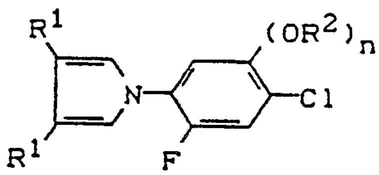

(I)

in der

$R^1$ Methyl oder eine aus zwei miteinander verbundenen Resten $R^1$ gebildete Tetramethylen-Gruppe bezeichnet,

$R^2$ Wasserstoff, $C_{1-6}$-Alkyl, Cyclopropylmethyl, $C_{1-4}$-Alkoxy-$C_{1-2}$-alkyl, $C_{1-4}$-Alkylthio-$C_{1-2}$-alkyl, $C_{1-4}$-Alkylsulfinyl-$C_{1-2}$-alkyl, $C_{1-4}$-Alkylsulfonyl-$C_{1-2}$-alkyl oder $R^2$ Benzyl oder Phenethyl, die gegebenfalls durch Halogen substituiert sind, $R^2$ weiterhin $C_{3-4}$ Alkenyl, $C_{3-4}$-Halogenoalkenyl, $C_{3-4}$-Alkinyl, $C_{2-3}$-Cyanoalkyl, Carbamoylmethyl, Thiocarbamoylmethyl, Trimethylsilylmethyl, $CH_2COOR^3$,

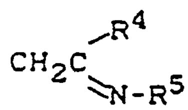

oder eine $C_{1-2}$-Alkyl-Gruppe, die mit einer gegebenenfalls substituierbaren 5- oder 6-gliedrigen heterocyclischen Gruppe verbunden ist, bezeichnet,

$R^3$ $C_{1-6}$-Alkyl oder $C_{5-6}$-Cycloalkyl bezeichnet,

$R^4$ Wasserstoff, Methyl, Ethyl oder Cyclopropyl bezeichnet,

$R^5$ $OR^6$ oder $N(CH_3)_2$ bezeichnet, worin

$R^6$ Wasserstoff, $C_{1-5}$-Alkyl, Allyl, Propargyl oder Benzyl bezeichnet, und

n 0 oder 1 darstellt.

2. Verbindungen der Formel (I) nach Anspruch 1, dadurch gekennzeichnet, daß

$R^1$ Methyl oder eine aus zwei miteinander verbundenen Resten $R^1$ gebildete Tetramethylen-Gruppe bezeichnet,

$R^2$ Wasserstoff, $C_{1-4}$-Alkyl, Cyclopropylmethyl, $C_{1-2}$-Alkoxy-$C_{1-2}$-alkyl, $C_{1-2}$-Alkylthio-$C_{1-2}$-alkyl, $C_{1-2}$-Alkylsulfinylmethyl, $C_{1-2}$-Alkylsulfonylmethyl Benzyl, $C_3$-Alkenyl, $C_3$-Halogenoalkenyl, Propargyl, Cyanomethyl oder eine Methyl-Gruppe, die mit einer fünfgliedrigen heterocyclischen Gruppe oder einer sechsgliedrigen aromatischen heterocyclischen Gruppe verbunden ist, die jede wenigstens ein Stickstoffatom enthalten, bezeichnet, wobei der fünfgliedrige und der sechsgliedrige Ring durch $C_{1-2}$-Alkyl oder $C_{1-2}$-Alkoxy substituiert sein können, und

n 0 oder 1 darstellt.

3. Verbindungen der Formel (I) nach Anspruch 1, dadurch gekennzeichnet, daß

$R^1$ Methyl oder eine aus zwei miteinander verbundenen Resten $R^1$ gebildete Tetramethylen-Gruppe bezeichnet,

$R^2$ n-Propyl, iso-Propyl, n-, iso-, sec- oder tert-Butyl, Cyclopropylmethyl, Methoxymethyl, Ethoxymethyl, Methylthiomethyl, Ethylthiomethyl, Benzyl, Allyl, 2-Chloroallyl, Propargyl, Cyanomethyl oder eine mit einer fünfgliedigen heterocyclischen Gruppe, die ein oder zwei Stickstoff-Atome und entweder ein Sauerstoff- oder ein Schwefel-Atom umfaßt, verbundene Methyl-Gruppe oder eine mit einer sechsgliedigen aromatischen heterocyclischen Gruppe, die ein oder zwei Stickstoff-Atome umfaßt, verbundene Methyl-Gruppe bezeichnet, wobei der fünfgliedrige und der sechsgliedrige Ring durch Methyl oder Methoxy substituiert sein können, und

n 1 darstellt.

4. Verbindungen der Formel (I) nach den Ansprüchen 1 bis 3, nämlich 2-(4-Chloro-2-fluoro-5-propargyloxyphenyl)-4,5,6,7-tetrahydroisobenzindol der Formel

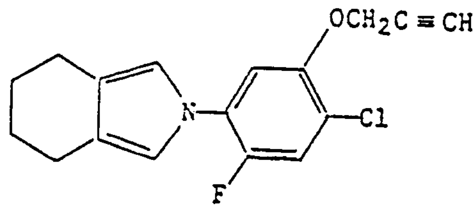

,

3,4-Dimethyl-1-(4-chloro-2-fluoro-5-propargyloxyphenyl)pyrrol der Formel

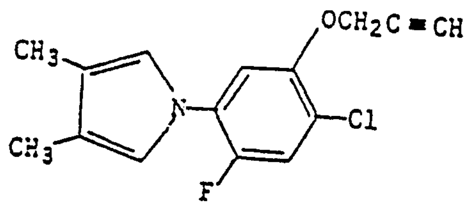

,

2-(5-Allyloxy-4-chloro-2-fluoro-phenyl)-4,5,6,7-tetrahydroisobenzindol der Formel

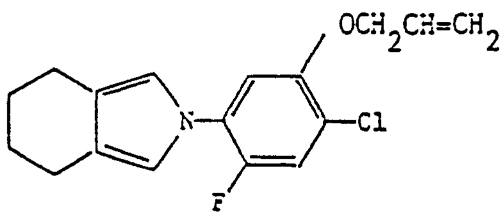

,

2-[4-Chloro-2-fluoro-5-(pyridin-2-yl-methoxy)phenyl]-4,5,6,7-tetrahydroisobenzindol der Formel

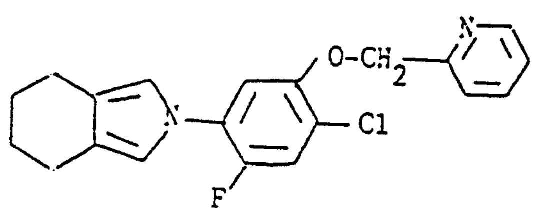

und
2-[4-Chloro-2-fluoro-5-(isoxazol-3-yl-methoxy)phenyl]-4,5,6,7-tetrahydroisobenzindol der Formel

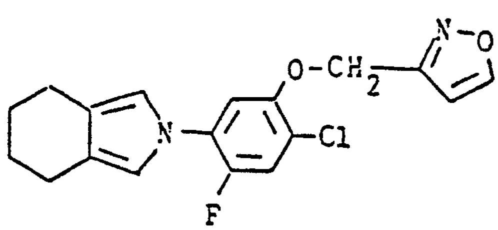

.

5. Verfahren zur Herstellung von 1 Phenylpyrrolen der Formel (I)

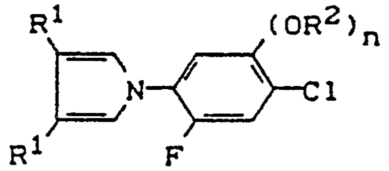

(I)

,

in der

$R^1$ Methyl oder eine aus zwei miteinander verbundenen Resten $R^1$ gebildete Tetramethylen-Gruppe bezeichnet,

$R^2$ Wasserstoff, $C_{1-6}$-Alkyl, Cyclopropylmethyl, $C_{1-4}$-Alkoxy-$C_{1-2}$-alkyl, $C_{1-4}$-Alkylthio-$C_{1-2}$-alkyl, $C_{1-4}$-Alkylsulfinyl-$C_{1-2}$-alkyl, $C_{1-4}$-Alkylsulfonyl-$C_{1-2}$-alkyl oder $R^2$ Benzyl oder Phenethyl, die gegebenenfalls durch Halogen substituiert sind, $R^2$ weiterhin $C_{3-4}$-Alkenyl, $C_{3-4}$-Halogenoalkenyl, $C_{3-4}$-Alkinyl, $C_{2-3}$-Cyanoalkyl, Carbamoylmethyl, Thiocarbamoylmethyl, Trimethysilylmethyl, $CH_2COOR^3$,

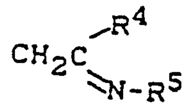

oder eine $C_{1-2}$-Alkyl-Gruppe, die mit einer gegebenenfalls substituierbaren 5- oder 6-gliedrigen heterocyclischen Gruppe verbunden ist, bezeichnet,

$R^3$ $C_{1-6}$-Alkyl oder $C_{5-6}$-Cycloalkyl bezeichnet,

$R^4$ Wasserstoff, Methyl, Ethyl oder Cyclopropyl bezeichnet,

$R^5$ $OR^6$ oder $N(CH_3)_2$ bezeichnet, worin

$R^6$ Wasserstoff, $C_{1-5}$-Alkyl, Allyl, Propargyl oder Benzyl bezeichnet, und

n 0 oder 1 darstellt,

dadurch gekennzeichnet, daß

a) Verbindungen der Formel (II)

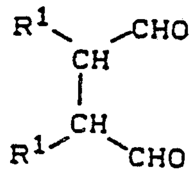

(II)

,

in der $R^1$ die gleichen Substituenten bezeichnet, wie sie im Vorstehenden genannt sind, mit Verbindungen der Formel (III)

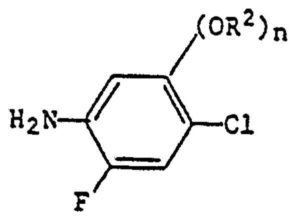

(III)

,

in der $R^2$ und n die gleichen Bedeutungen haben, wie sie im Vorstehenden angegeben sind, in Gegenwart inerter Lösungsmittel umgesetzt werden

oder

b) Verbindungen der Formel (IV)

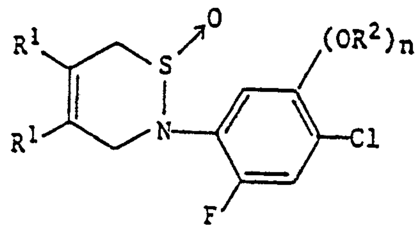

(IV)

,

in der $R^1$, $R^2$ und n die gleichen Bedeutungen haben, wie sie im Vorstehenden angegeben sind, einer Alkali-Behandlung in Gegenwart inerter Lösungsmittel unterworfen werden
oder

c) in dem Fall, in dem $R^2$ nicht Wasserstoff ist, Verbindungen der Formel (V)

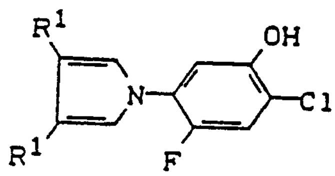

(V)

,

in der $R^1$ die gleichen Substituenten bezeichnet, wie sie im Vorstehenden genannt sind,
mit Verbindungen der Formel (VI)

$M - R^{2-1}$ (VI),

in der $R^{2-1}$ die gleichen Substituenten wie $R^2$ mit Ausnahme von Wasserstoff bezeichnet und M Halogen, Methansulfonyloxy, Tosyloxy oder $R^{2-1}$-$OSO_2$-O bezeichnet, worin $R^{2-1}$ die im Vorstehenden angegebenen Bedeutungen hat,
in Gegenwart inerter Lösungsmittel und gegebenenfalls in Gegenwart einer Base umgesetzt werden.

6. Herbizide Zusammensetzungen, dadurch gekennzeichnet, daß sie wenigstens ein 1-Phenylpyrrol der Formel (I) nach irgendeinem der Ansprüche 1 bis 4 enthalten.

7. Verfahren zur Unkrautbekampfung, dadurch gekennzeichnet, daß man 1-Phenylpyrrole der Formel (I) nach irgendeinem der Ansprüche 1 bis 4 auf Unkräuter und/oder ihren Lebensraum einwirken läßt.

8. Verwendung von 1-Phenylpyrrolen der Formel (I) nach irgendeinem der Ansprüche 1 bis 4 zur Unkrautbekämpfung.

9. Verfahren zur Herstellung herbizider Zusammensetzungen, dadurch gekennzeichnet, daß 1-Phenylpyrrole der Formel (I) nach irgendeinem der Ansprüche 1 bis 4 mit Streckmitteln und/oder grenzflächenaktiven Mitteln vermischt werden.

Europäisches Patentamt

**EUROPÄISCHER RECHERCHENBERICHT**

Nummer der Anmeldung

EP 89112245.9

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int Cl.4) |
| A | AT - B - 304 528 (CIBA-GEIGY AG) * Seite 2 * -- | 1,5 | C 07 D 207/325<br>A 01 N 43/34<br>C 07 D 209/44<br>C 07 D 401/12<br>C 07 D 413/12<br>C 07 D 417/12<br>C 07 D 403/12 |
| A | DE - A1 - 3 735 641 (CIBA-GEIGY AG) * Zusammenfassung * -- | 1 | |
| A | CHEMICAL ABSTRACTS, Band 68, Nr. 23, 3. Juni 1968, Columbus, Ohio, USA EDWARD E. GARCIA et al. "Synthesis of polycyclic fused (1,2-a) pyrroles" Seite 10157, Spalte 1, Zusammenfassung-Nr. 105 181s & J. Org. Chem. 33(4), 1359-63 (1968) (Eng). -- | 1,5 | |
| A | CHEMICAL ABSTRACTS, Band 84, Nr. 25, 21. Juni 1976, Columbus, Ohio, USA DORNAUER, HORST et al. "Spiro(pyrrolo(1,2-a)quinoxalines)" Seite 584, Spalte 1, Zusammenfassung-Nr. 180 293p & US-PS 3 939 159 -- | 1 | RECHERCHIERTE SACHGEBIETE (Int. Cl.4)<br>C 07 D 207/00<br>C 07 D 209/00<br>C 07 D 401/00<br>C 07 D 413/00<br>C 07 D 417/00<br>C 07 D 403/00 |
| A | CHEMICAL ABSTRACTS, Band 92, Nr. 19, 12. Mai 1980, Columbus, Ohio, USA WATANABE Y. "The reductive amination of phthalaldehyde by tetracarbonylhydridoferrate. Synthesis of 2-arylisoindoles" Seite 587, Spalte 1, Zusammenfassung-Nr. 163 800z & Tetrahedron 1979, 35(11), | 1 | |

Der vorliegende Recherchenbericht wurde für alle Patentansprüche erstellt.

| Recherchenort | Abschlußdatum der Recherche | Prüfer |
|---|---|---|
| VIENNA | 10-10-1989 | HEIN |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82

Europäisches Patentamt

**EUROPÄISCHER RECHERCHENBERICHT**

Nummer der Anmeldung

-2-
EP 89112245.9

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl 4) |
|---|---|---|---|
| | 1433-6 (Eng) -- | | |
| D,A | TETRAHEDRON, Band 23, 1967 Pergamon Press R.ALAN JONES et al. "Pyrrole studies - XII. The aromatic character of 1-substituted pyrroles" Seiten 4469-4479 -- | 1 | |
| D,A | JOURNAL OF CHEMICAL SOCIETY, Band 14, 1970 C.F.CANDY et al. "Pyrrole studies part XV. VILSMEIER--HAACK formylation of 1-substituted pyrroles" Seiten 2563-2567 -- | 1 | |
| D,A | JOURNAL OF HETEROCYCLIC CHEMISTRY, Band 9, Nr. 4, August 1972 H. EL KHADEM et al. "Synthesis and mass spectra of N-aryl-pyrroles and their chlorination products" Seiten 1413-1417 ---- | 1 | RECHERCHIERTE SACHGEBIETE (Int. Cl.4) |

Der vorliegende Recherchenbericht wurde für alle Patentansprüche erstellt

| Recherchenort | Abschlußdatum der Recherche | Prüfer |
|---|---|---|
| VIENNA | 10-10-1989 | HEIN |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument

EPA Form 1503 03 82